# EUROPEAN PATENT SPECIFICATION

(11) **EP 3 095 392 B1**
(45) Date of publication and mention of the grant of the patent: **01.11.2017**
(21) Application number: 15001478.5
(22) Date of filing: 18.05.2015
(51) Int. Cl.: A61B 17/06

(54) **PACKAGE FOR SUTURES**
VERPACKUNG FÜR NAHTFÄDEN
EMBALLAGE POUR SUTURES

(43) Date of publication of application: 23.11.2016
(73) Proprietor: DS-Technology GmbH, 71364 Winnenden (DE)
(72) Inventor: Dey, Clifford, 71573 Allmersbach im Tal (DE)
(74) Representative: Schmid, Barbara

(56) References cited:
- EP-A1- 2 172 157
- WO-A1-2013/049400
- GB-A- 2 514 994
- US-A- 5 230 424

## Description

### TECHNICAL FIELD

This invention relates to packages for surgical sutures. Conventional surgical suture and needle packages serve several useful functions, including protecting the needles and sutures during handling, shipping, and storage. In addition, the packages facilitate access and release of the needles and sutures during surgery or other medical procedures prior to application. The packages may be used for surgical sutures armed with surgical needles or for unarmed surgical sutures without needles.

### PRIOR ART

Packaging for surgical sutures with or without needles is well known in the art. There are two types of packages that have been conventionally used for surgical needles and sutures. One type of package is a paper folder package wherein a medical grade paperboard is folded and cut into a plurality of panels. The suture is then wound onto a panel, and the package is then assembled by first folding the panels into a desired configuration, and then locking the panels in place using slits and locking tabs which have been cut into the panels.

Another type of suture package which has been used is a tray package having a winding channel. These tray packages typically have an oval shape with outer and inner walls forming an oval winding channel. The packages are typically molded from plastics. The packages are mounted onto a winding fixture and sutures are then wound into the winding channel. Suture packages typically have a needle park member for mounting and securing a surgical needle if a surgical needle is mounted to the sutures. Conventional needle parks can consist of foam members, or equivalent retention structures. The needle park members can also be utilized for mounting one end of a suture wound into the winding channel.

U.S. Patent No. 4,961,498 discloses a two-piece suture package having an oval winding channel. U.S. Patent No. 4,967,902 discloses a one-piece channel suture package having a plurality of door members which retain the suture in the channel. U.S. Patent No. 5,230,424 discloses a package having a substantially square shape and having a square shaped suture channel wherein a plurality of cantilevered doors are mounted to an inner wall to maintain sutures in the channel. U.S. Patent No. 5,655,652 discloses a package having an oval-shaped winding channel with a top friction plate member in lieu of doors or cantilevered doors.

U.S. Patent No. 5,131,533 discloses a needle park having a hinged section. U.S. Patent No. 5,180,053 discloses a suture package having a cantilevered arm needle park. This park extends vertical to the base of the package. This type of park has the disadvantage of allowing the needle to release if the package is flexed during transportation.

U.S. Patent No. 6,135,272 discloses a plurality of cantilevered cover door members with spaces in between. These door members have a disadvantage of deforming if the stylus is moving at a high speed therefore limiting the winding speed.

WO 2013/049400 A1 discloses a suture package having two halves. The interior of the body portion is provided with a pair of posts in one half of the body portion and a corresponding pair of mating bosses in the other half of the body portion. When the two halves of the base are pressed together, the posts fit into the bosses in a press fit or snap fit manner to secure the two halves together. The posts and bosses also provide a structure around which the suture strands may be wrapped.

EP 2 172 157 A1 discloses a suture package for retaining a barbed suture including a suture retaining member with an outer wall and an inner wall. The inner wall is radially spaced from the outer wall and defines a suture retaining area therebetween. The outer wall includes a plurality of inwardly extending tabs configured to engage a cover. The suture package further includes a cover configured to be received within the outer wall of the suture retaining member and to selectivly engage the inwardly extending tabs formed thereon.

Although the suture tray packages of the prior art are adequate and effective for their intended use, there are disadvantages associated with such packages. An example of one type of problem which may occur is suture "hang-up" when the surgeon attempts to withdraw the suture from the package. Accordingly, there is a need in this art for novel suture tray packages having winding channels which are readily adaptable to high-speed packaging processes which overcome the disadvantages of the prior art packages, including problems associated with suture withdrawal. Accordingly there is a need for gasses to be able to flow easily around the sutures to facilitate sterilization and antimicrobial coating.

### SUMMARY OF THE INVENTION

Proceeding from this previously known prior art, it is an object of the present invention to provide a novel tray package having a winding channel which is useful in a high-speed packaging process for packaging surgical sutures.

It is also an object of the present invention to provide a tray package with the ability to securely maintain sutures in a channel that has an open interior wall to facilitate application of sterilization/antibacterial application.

It is still yet a further object of the present invention to provide a novel suture tray package which facilitates withdrawal of sutures from the package.

It is still yet a further object of the present invention to provide a novel needle park to provide better retention of the needles during handling.

It is still yet a further object of the present invention to provide a novel suture channel that allows for unobstructed flow of sterilization and/or antimicrobial coating gas.

The suture package according to the invention is produced by the features of the main claim. Appropriate developments of the invention are subject matter of further claims following the main claim.

Accordingly, a suture package is disclosed. The package has a base member having a top surface, a bottom surface, an outer periphery and a longitudinal axis. An outer wall extends upwardly about the
periphery of said base member, said outer wall having an inner surface, an outer surface, and a top. An inner plurality of cylindrical standoff members is arranged in an oval shape that defines and captures the suture therefore forming a channel for the suture to lie in. These standoff members have an interference fit snap that allows the cover member to be secured.

There is also a paper suture channel cover member for mounting to the base member. The suture channel cover member has a top surface, a bottom surface and an outer periphery. A plurality of fastener holes exists in the cover member. A port exit opening having a first end and a second end is located in the outer track wall and forms a suture port.

A suture park member extends up from the base member to the bottom surface of the cover member. A slit in the base member interior to the outer wall forms the tab lifting member. The cover member is mounted to the base member to form the package of the present invention by aligning the cover member fastener holes with the base member standoff members and inserting the interference fit snaps of the base member into the holes in the cover. This forms a suture channel between the inner surface of the outer wall of the base member, the top surface of the base member, the outer side of the standoff members and the bottom surface of the suture cover member. An optional plastic top suture cover may be utilized to complete assembled package. This assembly is completed after the suture is wound into the channel formed by the cover top, the base outer wall, the base top surface and the suture winding pins.

These and other features and advantages of the present invention will become more apparent from the following description and accompanying drawings.

Further advantages and features of the invention can be gathered from the features which are specified further in the claims and from the following exemplary embodiment.

### BRIEF DESCRIPTION OF THE DRAWINGS

In the following text, the invention will be described and explained in greater detail using the exemplary embodiment which is shown in the drawings.
- Fig. 1: is a perspective view of a package of the present invention having a needle mounted therein.
- Fig. 2: is an exploded side view of the package of Fig. 1.
- Fig. 3: is a top, plan view of the package of Fig. 1.
- Fig. 4: is a top view of the base member of the package in Fig. 1.
- Fig. 5: is a bottom view of the base member of the package in Fig. 1.
- Fig. 6: is an enlarged view of the base member of the package in Fig. 1.
- Fig. 7: is a perspective enlarged view of the base of Fig. 4 showing the needle park with needle.
- Fig. 8: is a top view of the suture cover of the package of Fig. 1.
- Fig. 9: is a cut away enlarged view showing the suture cover member in attached position.
- Fig. 10: is a perspective view showing suture winding pins engaged with the suture and needle.

### DESCRIPTION OF THE PREFERRED EMBODIMENTS

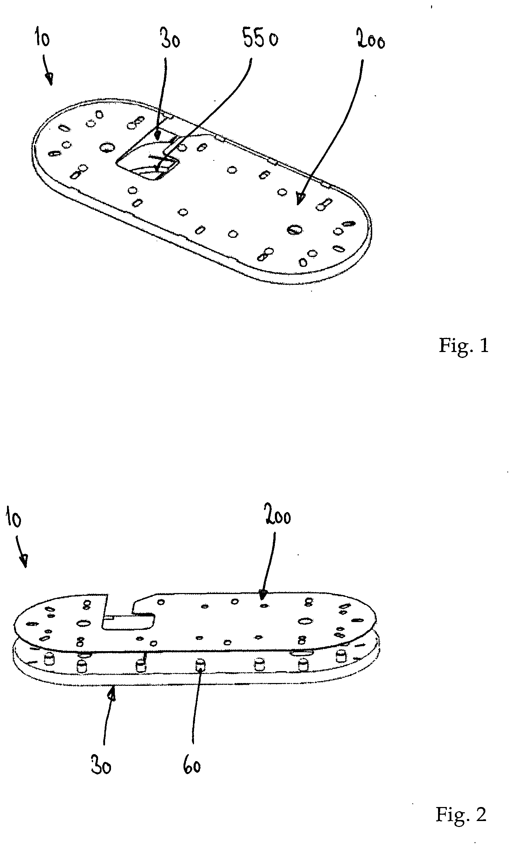

The package 10 of the present invention is illustrated in Figs. 1 to 10. As seen in Figs. 1,2, and 3, the package 10 has a base member 30 and a suture channel cover member 200.

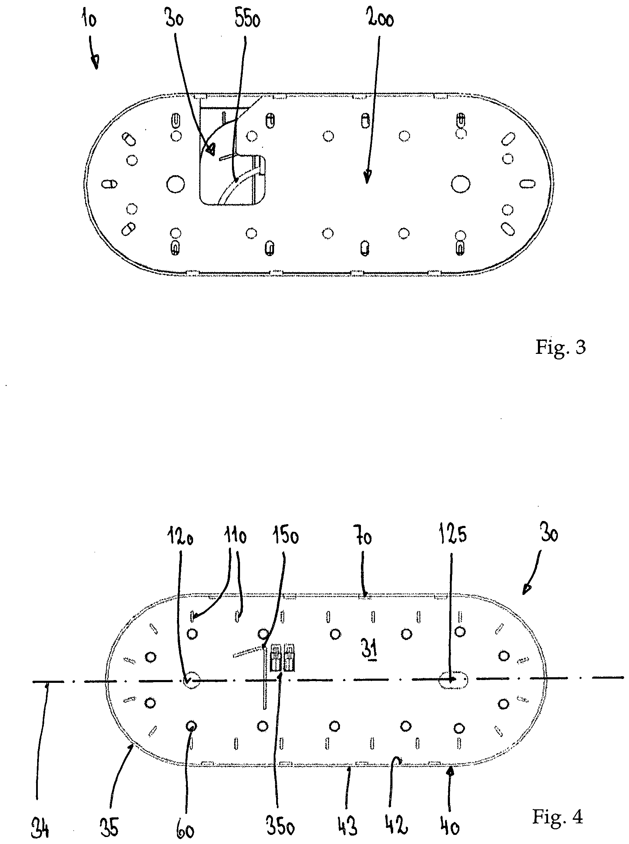

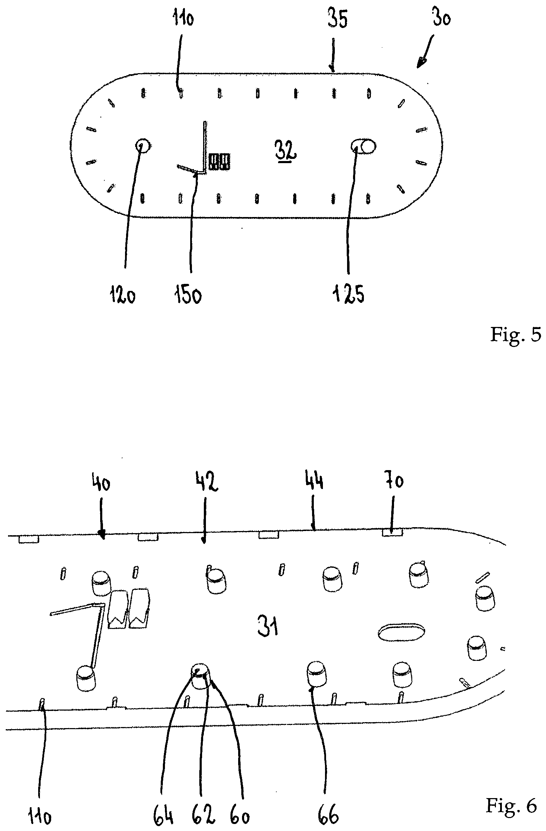

Referring now in more detail to Figs. 4 and 5, the base member 30 is seen to have a top surface 31 and a bottom surface 32. The base member 30 is also seen to have an outer periphery 35. The base member 30 is seen to be a substantially flat and substantially oval shaped member having a longitudinal axis 34. However, although it is desired that the base member 30 along with the package 10 be oval shaped, other configurations can be used including circular, polygonal, square with rounded corners, and the like and combinations thereof and equivalents thereof.

Extending upwardly about the periphery 35 of the base member 30 is an outer wall 40. Outer wall 40 is seen to have a bottom, an inner surface 42, an outer surface 43 and a top 44.

In Fig. 2 the standoff members 60 are seen to extend upward from the top surface 31 of the base member 30. According to Fig. 6 the standoff members 60 have flat tops 62. These flat tops 62 have an interference fit snap 64 for the attachment of the cover member 200. The shape of the snap 64 is shown in its preferred configuration. Alternatively, the snap can have a geometric shape, or any shape that provides an interference fit. The tops of the snaps 64 are preferably below the top 44 of the outer wall 40. The standoff members 60 are seen to preferably have a cylindrical surface 66, but they may also have flat outer surfaces. If desired, the standoff members 60 may have other configurations for outer surfaces including semi-circular, polygonal, oval, triangular, or combinations thereof and equivalents thereof and the like. A combination of standoff members 60 with flat and with curved outer surfaces are also possible.

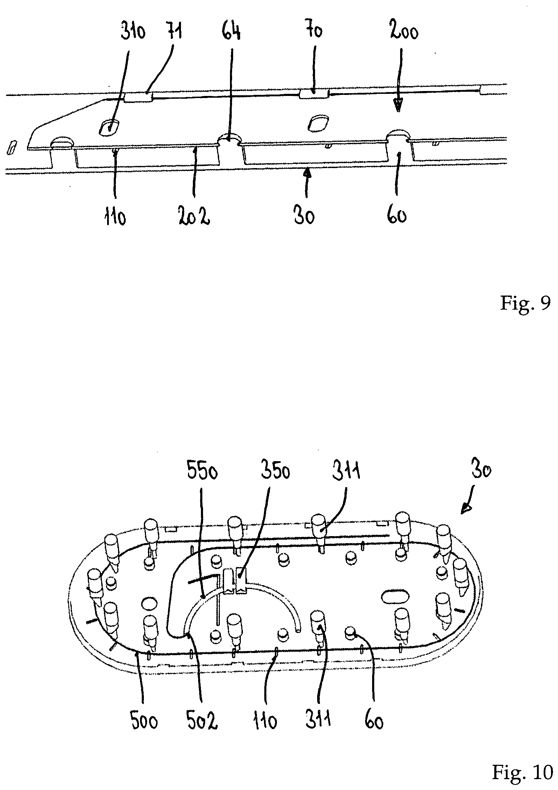

There are a multiple of outer cover locks 70 which can be seen in Fig. 6 and 9. The cover locks 70 have a top 71, a bottom, a right side and a left side. The cover locks 70 hold the cover member 200 in place after winding is complete.

Extending through the bottom of the base member 30 are a plurality of air slots 110 (see Fig. 4). Also extending through the bottom of the base member 30 are the circular winding drive pin locating hole 120 and the oval drive pin locating hole 125. The holes 120 and 125 are seen to be disposed along the longitudinal axis 34 and are at opposite ends of the base member 30. The lifting tab 150 is seen to be located in the base member 30 toward the circular locating hole 120.

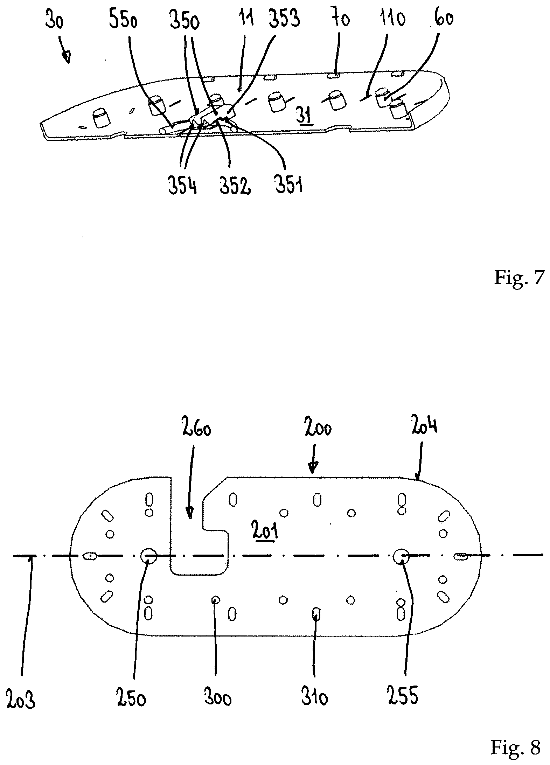

Referring now to Fig. 1 and 8, the suture channel cover 200 is seen to be illustrated. The suture channel cover 200 has a top surface 201, a bottom surface 202 and a periphery 204. The pin winding holes 250 and 255 are seen to be contained at opposite ends of the cover member 200. Pin winding holes 250, 255 are seen to extend through the cover member 200 and to be disposed in the cover member 200 along its longitudinal axis 203, toward either end, and are further seen to line up with winding drive pin holes 120 and 125 in the base member 30. Pin winding holes 250 and 255 are seen to be circular in shape. However, other geometric shapes can be utilized.

The suture exit port 260 is seen to be contained in the cover member 200. Also seen to extend through the cover member 200 are the cover snap mounting holes 300 that mate up with the standoff members 60 of the base member 30. The diameter and shape is determined by the type of interference fit and the style of the snaps 64.

There is a multiplicity of suture winding pin holes 310 that extend through the cover member 200. The preferred shape is oval but other shapes can be utilized such as round, octagonal, semi-circular, polygonal, triangular, combinations thereof and equivalents thereof and the like.

The needle park in Fig. 7 is shown in its preferred configuration with two horizontal cantilevered members 350. Each member 350 has a V-shaped notch 354 that leaves a small land to contact the needle 550 and hold it in place. This land then deforms during the winding process due to pressure from the winding head creating a hold area 353. Fig. 7 shows two needle park areas, a large one 352 and a small one 351. It is understood that there can be more than two steppings from largest to smallest needle front to back.

The packages 10 of the present invention are assembled in the following manner. In order to mount sutures 500 having surgical needles 550 mounted to one end 502 in the package 10, the base member 30 is mounted into a conventional, rotatable winding fixture, such that the winding pins 311 of the winding fixture are inserted through the winding drive pin openings 125 and 120. The needle 550 is placed into the needle park members 350. Then, the suture 500 is threaded out of port 260 into the channel 11. Next, the suture channel cover 200 is printed by conventional means and pin winding holes 250 and 255 of the suture cover member 200 are aligned with winding drive openings 125 and 120 of the base member 30. The suture cover 200 is then brought down so that the bottom surface 202 is less than one millimeter above the top 44 of the outer wall 40 of the base member 30. The suture winding pins 311 in the winding now come through the holes 310 in the suture cover member 200 and rest on the top surface 31 of the base member 30. This forms a suture channel for winding formed by the top surface 31 of the base member 30, the suture winding pins 311, and the bottom surface 202 of the cover member 200. This creates a small gap for the suture 500 to enter the track area 11.

Alternately the suture cover 200 can be brought down and the interference fit snaps 64 are pushed through the interference fit holes 300 in the suture cover member 200 until the flat tops 62 of the standoff members 60 contact the bottom surface 202 of the suture cover 200 and held in place by the winding head. The bottom surface 202 of the suture cover 200 should be left in contact with the top 71 of the outer cover locks 70 allowing the suture cover 200 to be lifted to wind the suture 500 into the suture track 11.

Next, the suture 500 is guided into the package 10 by a conventional stylus which lifts the cover member 200 or runs between the suture cover 200 and the top 44 of the outer wall 40 of the base member. Package 10 is rotated in the fixture such that the suture 500 is completely wound in the suture track 11 as the package is rotated. This may be repeated with additional sutures 500 and needles 550. After winding is finished the suture cover 200 is pushed into the fully locked position, where the top surface 201 of the cover member 200 is in contact with the bottom of the outer cover locks 70 and the interference fit snaps 64 are pushed through the interference fit holes 300 in the cover member 200. The package 10 containing the wound sutures 500 and needles 550 may then be placed in a conventional pouch or package for conventional sterilization treatments such as gaseous sterilants, autoclaving, radiation and the like.

This invention will allow for high speed winding due to its capability to be wound before the cover member 200 is locked in place.

When used by the physician in a surgical procedure, the package 10 is placed into a sterile field. Using a conventional needle grasper, the surgeon pushes down lifting tab 150 and the needle 550 is grasped and removed from the needle park members 350. The needle 550 and the suture 500 are then pulled away from the package 10 and suture 500 exits through exit port 260 and channel exit and then from channel 11.

The packages 10 of the present invention may be manufactured from conventional moldable materials. It is especially preferred to use polyolefin materials such as polyethylene and polypropylene, other thermoplastic materials, and polyester materials such as nylon, and equivalents thereof. Preferably the packages 10 of the present invention are injection molded, however, the packages 10 may be formed by other conventional processes and equivalents thereof including thermo-forming. If desired, the packages 10 may be manufactured as individual assemblies or components which are then assembled.

The sutures 500 and needles 550 that can be packaged in the packages 10 of the present invention include conventional surgical needles and conventional bioabsorbable and nonabsorbable surgical sutures and equivalents thereof. The packages 10 of the present invention are useful to package small diameter sutures which were previously difficult to package in tray packages because of removal or hang-up problems upon withdrawal of such suture from the packages. These problems have been overcome using the packages 10 of the present invention.

## Claims

1. A package (10) for surgical sutures (500), comprising
- a base member (30) having a top surface (31), a bottom surface (32), an outer periphery (35) and a longitudinal axis (34),
- an outer wall (40) extending upwardly from the periphery (35) of said base member (30), said outer wall (40) having an inner surface (42), an outer surface (43), and a top (44),
- a plurality of lock members (70) extending inward from the inner surface (42) of the outer wall (40), each lock member (70) having a top (71), a bottom and an outer surface,
- a plurality of cylindrical standoff members (60) arranged in an oval shape that defines and captures the suture (500), said standoff members (60) extending from the inner surface of the base member (30), each standoff member (60) having a top (62), a bottom and an outer surface (66), said standoff members (60) in conjunction with each other forming a suture track to retain suture (500),
- a suture channel cover member (200) having a top surface (201), a bottom surface (202) and an outer periphery (204),
- an opening (260) in the outer edge of the cover member (200) forming a suture port having a first end and a second end,
- needle park means (350) located interior to the cylindrical standoff members (60) and extending from the top surface (31) of the base member (30) for retaining a surgical needle (550),
- **characterized in that**, said package (10) comprises a plurality of shaped snaps (64) to accept the cover member (200), each shaped snap (64) placed on the top (62) of a standoff member (60),
- said cover member (200) having a plurality of precision holes (300) extending through the cover member (200) to lock said cover member (200) to the shaped snaps (64) of the standoff members (60) by pushing the shaped snaps (64) through the precision holes (300) in the cover member (200) until the top (62) of a standoff member (60) is in contact with the bottom surface (202) of the cover member (200) and the bottom of the shaped snap (64) is in contact with the top surface (201) of the cover member (200).

2. Package according to claim 1
additionally comprising
- holes (250, 255) through the cover member (200) for receiving winding pins.

3. Package according to claim 1 or 2
further comprising
- a plurality of air slots (110) in a suture track area (11) of the base member (30).

4. Package according to one of the preceding claims
further comprising
- a suture (500) wound into a winding channel (11),
- a surgical needle (550) mounted in the needle park means (350).

5. Package according to one of the preceding claims
wherein
- the shaped snaps (64) are snap fit or heat deformed through the cover member (200).

6. Package according to one of the preceding claims
wherein
- the package (10) has an oval configuration.

7. Package according to one of the preceding claims
wherein
- the needle park member comprises a horizontal member (350), with multiple elevations (351, 352) from the top surface (31) of the base member (30),
- the bottom of the horizontal member (350) is at least partially located over an opening (150) in the base member (30).

8. Package according to claim 7
wherein
- the bottom of the horizontal park member (350) has thin vertical walls to allow for deformation after a needle (550) is parked to provide locking of the needle (550).

9. Package according to one of the preceding claims
- having an open interior wall to the suture track formed by the cylindrical standoff members (60) allowing for sterilization/antibacterial gas to freely move around suture.

## Patentansprüche

1. Verpackung (10) für chirurgische Nahtfäden (500), umfassend
- ein Basiselement (30) mit einer oberen Fläche (31), einer unteren Fläche (32), einem Außenumfang (35) und einer Längsachse (34),
- eine Außenwand (40), die sich vom Umfang (35) des Basiselements (30) nach oben erstreckt, wobei die Außenwand (40) eine Innenseite (42), eine Außenseite (43) und eine Oberseite (44) aufweist,
- eine Vielzahl von Verriegelungselementen (70), die sich von der Innenseite (42) der Außenwand (40) einwärts erstrecken, wobei jedes Verriegelungselement (70) eine Oberseite (71), eine Unterseite und eine Außenseite aufweist,
- eine Vielzahl von zylindrischen Abstandselementen (60), die in einer ovalen Gestalt angeordnet sind, die den Nahtfaden (500) definiert und einfängt, wobei sich die Abstandselemente (60) von der Innenseite des Basiselements (30) erstrecken, wobei jedes Abstandselement (60) eine Oberseite (62), eine Unterseite und eine Außenseite (66) aufweist, wobei die Abstandselemente (60) in Verbindung miteinander eine Nahtbahn zum Festhalten des Nahtfadens (500) bilden,
- ein Nahtkanalabdeckelement (200) mit einer oberen Fläche (201), einer unteren Fläche (202) und einem Außenumfang (204),
- eine Öffnung (260) im Außenrand des Abdeckelements (200), die eine Nahtfadenöffnung mit einem ersten Ende und einem zweiten Ende bildet,
- Nadelparkmittel (350), die auf der Innenseite des zylindrischen Abstandselements (60) angeordnet sind und sich von der oberen Fläche (31) des Basiselements (30) zum Festhalten einer chirurgischen Nadel (550) erstrecken,
**dadurch gekennzeichnet, dass** die Verpackung (10)
- eine Vielzahl von geformten Schnappelementen (64) zur Aufnahme des Abdeckelements (200) umfasst, wobei jedes geformte Schnappelement (64) auf der Oberseite (62) eines Abstandselements (60) platziert ist,
- das Abdeckelement (200) eine Vielzahl von Präzisionslöchern (300) aufweist, die sich durch das Abdeckelement (200) erstrecken, um das Abdeckelement (200) an den geformten Schnappelementen (64) der Abstandselemente (60) zu verriegeln, indem die geformten Schnappelemente (64) durch die Präzisionslöcher (300) im Abdeckelement (200) geschoben werden, bis die Oberseite (62) eines Abstandselements (60) mit der unteren Fläche (202) des Abdeckelements (200) in Berührung ist und die Unterseite des geformten Schnappelements (64) mit der oberen Fläche (201) des Abdeckelements (200) in Berührung ist.

2. Verpackung nach Anspruch 1, zusätzlich umfassend
- Löcher (250, 255) durch das Abdeckelement (200) zur Aufnahme von Aufwickelstiften.

3. Verpackung nach Anspruch 1 oder 2, ferner umfassend
- eine Vielzahl von Luftschlitzen (110) in einer Nahtbahnfläche (11) des Basiselements (30).

4. Verpackung nach einem der vorhergehenden Ansprüche, ferner umfassend
- einen Nahtfaden (500), der in einen Wickelkanal (11) gewickelt ist,
- eine chirurgische Nadel (550), die in dem Nadelparkmittel (350) angebracht ist.

5. Verpackung nach einem der vorhergehenden Ansprüche, wobei
- die geformten Schnappelemente (64) durch das Abdeckelement (200) eingeschnappt oder hitzeverformt werden.

6. Verpackung nach einem der vorhergehenden Ansprüche, wobei
- die Verpackung (10) eine ovale Konfiguration aufweist.

7. Verpackung nach einem der vorhergehenden Ansprüche, wobei
- das Nadelparkelement ein horizontales Element (350) mit mehreren Erhebungen (351, 352) von der oberen Fläche (31) des Basiselements (30) umfasst,
- die Unterseite des horizontalen Elements (350) mindestens teilweise über einer Öffnung (150) im Basiselement (30) angeordnet ist.

8. Verpackung nach Anspruch 7, wobei
- die Unterseite des horizontalen Parkelements (350) dünne vertikale Wände aufweist, um eine Verformung nach dem Parken einer Nadel (550) zu gestatten, damit die Nadel (550) verriegelt werden kann.

9. Verpackung nach einem der vorhergehenden Ansprüche,
- mit einer offenen Innenwand zur Nahtbahn, die durch die zylindrischen Abstandselemente (60) ausgebildet wird und es gestattet, dass Sterilisations-/antibakterielles Gas frei um den Nahtfaden fließt.

## Revendications

1. Emballage (10) pour des sutures chirurgicales (500), comprenant
un élément de base (30) présentant une surface supérieure (31), une surface inférieure (32), une périphérie extérieure (35) et un axe longitudinal (34),
une paroi extérieure (40) qui s'étend vers le haut à partir de la périphérie (35) dudit élément de base (30), ladite paroi extérieure (40) présentant une surface intérieure (42), une surface extérieure (43), et un sommet (44),
une pluralité d'éléments de verrouillage (70) qui s'étendent vers l'intérieur à partir de la surface intérieure (42) de la paroi extérieure (40), chaque élément de verrouillage (70) présentant un sommet (71), un pied et une surface extérieure,
une pluralité d'éléments de picot cylindriques (60) disposés en une forme ovale qui définit et capture la suture (500), lesdits éléments de picot (60) s'étendant à partir de la surface intérieure de l'élément de base (30), chaque élément de picot (60) présentant un sommet (62), un pied et une surface extérieure (66), lesdits éléments de picot (60) en conjonction les uns avec les autres formant une piste de suture pour retenir la suture (500),
un élément de recouvrement de canal de suture (200) présentant une surface supérieure (201), une surface inférieure (202) et une périphérie extérieure (204),
une ouverture (260) dans le bord extérieur de l'élément de recouvrement (200) formant un port de suture présentant une première extrémité et une seconde extrémité,
des moyens de rangement d'aiguille (350) situés à l'intérieur des éléments de picot cylindriques (60) et qui s'étendent à partir de la surface supérieure (31) de l'élément de base (30) pour retenir une aiguille chirurgicale (550),
**caractérisé en ce que** ledit emballage (10) comprend:
une pluralité de fermoirs profilés (64) pour accepter l'élément de recouvrement (200), chaque fermoir profilé (64) étant placé sur le sommet (62) d'un élément de picot (60),
ledit élément de recouvrement (200) comportant une pluralité de trous de précision (300) qui s'étendent à travers l'élément de recouvrement (200) afin de verrouiller ledit élément de recouvrement (200) sur les fermoirs profilés (64) des éléments de picot (60) en poussant les fermoirs profilés (64) à travers les trous de précision (300) dans l'élément de recouvrement (200) jusqu'à ce que le sommet (62) d'un élément de picot (60) soit en contact avec la surface inférieure (202) de l'élément de recouvrement (200) et que la base du fermoir profilé (64) soit en contact avec la surface supérieure (201) de l'élément de recouvrement (200).

2. Emballage selon la revendication 1, comportant en outre des trous (250, 255) à travers l'élément de recouvrement (200) destinés à recevoir des broches d'enroulement.

3. Emballage selon la revendication 1 ou 2, comportant en outre une pluralité de fentes d'aération (110) dans une région de piste de suture (11) de l'élément de base (30).

4. Emballage selon l'une quelconque des revendications précédentes, comprenant en outre:
une suture (500) enroulée dans un canal d'enroulement (11), et
une aiguille chirurgicale (550) montée dans les moyens de rangement d'aiguille (350).

5. Emballage selon l'une quelconque des revendications précédentes, dans lequel les fermoirs profilés (64) sont ajustés par pression ou déformés thermiquement à travers l'élément de recouvrement (200).

6. Emballage selon l'une quelconque des revendications précédentes, dans lequel l'emballage (10) présente une configuration ovale.

7. Emballage selon l'une quelconque des revendications précédentes dans lequel
l'élément de rangement d'aiguille comprend un élément horizontal (350), qui présente de multiples élévations (351, 352) à partir de la surface supérieure (31) de l'élément de base (30), et
le pied de l'élément horizontal (350) est au moins partiellement situé au-dessus d'une ouverture (150) dans l'élément de base (30).

8. Emballage selon la revendication 7, dans lequel le pied de l'élément de rangement horizontal (350) présente des parois verticales minces destinées à permettre une déformation après qu'une aiguille (550) ait été rangée de manière à réaliser le verrouillage de l'aiguille (550).

9. Emballage selon l'une quelconque des revendications précédentes, présentant une paroi intérieure ouverte au niveau de la piste de suture formée par les éléments de picot cylindriques (60) qui permet qu'un gaz de stérilisation/antibactérien puisse circuler librement autour de la suture.
